(19) Europäisches Patentamt
European Patent Office
Office européen des brevets

(11) Veröffentlichungsnummer : **0 036 511**
**B1**

(12) # EUROPÄISCHE PATENTSCHRIFT

(45) Veröffentlichungstag der Patentschrift : **26.09.84**

(21) Anmeldenummer : **81101480.2**

(22) Anmeldetag : **02.03.81**

(51) Int. Cl.³ : **C 07 C 31/18**, C 07 C 29/14, C 07 C 29/136, C 08 G 18/32, C 08 G 18/40, C 08 G 63/02, C 08 G 65/28, B 01 J 20/34// C07C69/00

(54) **Verfahren zur Herstellung von Gemischen von niedermolekularen mehrwertigen Alkoholen.**

(30) Priorität : **14.03.80 DE 3009847**

(43) Veröffentlichungstag der Anmeldung : **30.09.81 Patentblatt 81/39**

(45) Bekanntmachung des Hinweises auf die Patenterteilung : **26.09.84 Patentblatt 84/39**

(84) Benannte Vertragsstaaten : **BE CH DE FR GB IT LI SE**

(56) Entgegenhaltungen :
**EP-A- 0 002 473**
**EP-A- 0 007 100**
**EP-A- 0 007 102**
**DE-A- 888 096**
**US-A- 2 272 378**

(73) Patentinhaber : **BAYER AG**
**Konzernverwaltung RP Patentabteilung**
**D-5090 Leverkusen 1 Bayerwerk (DE)**

(72) Erfinder : **Richter, Roland, Dr.**
**Burgstrasse 43**
**D-5090 Leverkusen (DE)**
Erfinder : **Müller, Hanns Peter, Dr.**
**St.-Pankratius-Strasse 2**
**D-5068 Odenthal (DE)**
Erfinder : **Wagner, Kuno, Dr.**
**Am Kiesberg 8**
**D-5090 Leverkusen (DE)**
Erfinder : **Helm, Rudolf, Dr.**
**August-Clostermann-Strasse 6**
**D-5060 Bergisch Gladbach (DE)**
Erfinder : **Zander, Jürgen, Dr.**
**Tannenweg 5**
**D-5090 Leverkusen (DE)**

**EP 0 036 511 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von Gemischen von niedermolekularen mehrwertigen Alkoholen durch katalytische Hydrierung eines Gemisches verschiedener niedermolekularer Hydroxyaldehyde, Hydroxyketone und mehrwertiger Alkohole, welches bei der Kondensation von Formaldehyd entsteht und im folgenden als « Formose » bezeichnet wird.

Seit den Arbeiten von Butlerow und Loew (Ann. *120*, 295 (1861) und J. prakt. Chem. *33*, 321 (1886)) im vorigen Jahrhundert ist es bekannt, daß sich bei der Selbstkondensation des Formaldehydhydrats (Formosesynthese) Hydroxyaldehyde und Hydroxyketone bilden.

Formose kann beispielsweise durch Kondensation von Formaldehyd in Gegenwart von Blei-(II)-Verbindungen und von zur Endiolbildung befähigten Verbindungen bei einer Temperatur von 70 bis 110 °C und pH-Kontrolle durch Zusatz einer anorganischen oder organischen Base hergestellt werden (DE-OS 26 39 084). Aber auch andere Gemische von niedermolekularen Hydroxyaldehyden und/oder Hydroxyketonen und gegebenenfalls mehrwertigen Alkoholen sind im erfindungsgemäßen Verfahren einsetzbar.

Man war schon frühzeitig bemüht, die Gemische aus Hydroxyaldehyden, Hydroxyketonen und gegebenenfalls Polyalkoholen durch Reduktion der Carbonylgruppen in (farb)-stabile Gemische von Polyalkoholen umzuwandeln, die vielerlei Verwendungszwecken zugeführt werden können. Derartige, durch Reduktion von Formosen erhaltene Polyolgemische werden im folgenden « Formite » genannt.

So arbeitet man bei den ältesten Verfahren mit Na-Amalgam (vgl. Loew, J. prakt. Chem. *33*, 325 (1886)), bei neueren dagegen mit Natriumborhydrid (vgl. R.D. Partridge, A.H. Weiss und D. Todd, Carbohydrate Research *24*, 42 (1972)). Die Reduktion kann aber z. B. auch auf elektrochemischem Weg erfolgen.

Weiterhin werden Verfahren, wie man sie zur katalytischen Hydrierung von Zuckern anwendet, in abgewandelter Form auf die Formose-Reduktion übertragen. Dabei werden je nach Verfahrensweise sehr unterschiedliche Bedingungen angewandt, insbesondere was die Art und Menge des Katalysators und die Konzentration der eingesetzten Formose betrifft. Fast allen diesen Verfahren ist aber eigen, daß sie wirtschaftlich in jeder Hinsicht unbefriedigend sind. So beschreiben z. B. L. Orthner und E. Gerisch (Biochem. Zeitung *259*, 30 (1933)) ein Verfahren zur katalytischen Hydrierung von Formose, bei dem in 7-8 stündiger Reaktion bei 130 °C und 120 bar Wasserstoffdruck eine 4 %ige wäßrige Formoselösung mit 170 Gew.-%, bezogen auf Formose, an Raney-Nickel hydriert wird. Außerdem wurde bei den früheren Verfahren, die Metall- oder Edelmetallkatalysatoren, insbesondere Raney-Nickel, einsetzen, strikt vermieden, im alkalischen Bereich zu hydrieren, da man befürchtete, Nebenreaktionen und damit verbundene Verfärbungen hervorzurufen (vgl. W. Langenbeck, J. f. prakt. Chemie *3*, 206 (1956)), obwohl gerade Raney-Nickel im alkalischen Milieu erst seine volle Aktivität entfaltet.

Die teilweise sehr extremen Bedingungen und die damit verbundenen Nachteile aller bekannten Hydrierverfahren für Formose haben ihre Ursache darin, daß es infolge der außerordentlichen Polymolekularität der Formose nicht möglich ist, bekannte Verfahren, wie sie z. B. zur katalytischen Hydrierung von D-Glucose zu Sorbit durchgeführt werden, auf die Formose-Hydrierung zu übertragen. Die Hydrierungsmethoden von Zuckern versagen wegen der Nichtvergleichbarkeit bei dem Versuch ihrer Übertragung auf Formose.

Ein weiteres Merkmal und ein damit verbundener Nachteil aller bisherigen Hydrierverfahren liegt in der Herstellungsweise der Formose begründet. Bei den Standardverfahren zur Darstellung von Formose werden basische anorganische Salze als Katalysatoren verwendet, sehr häufig Ca- und Pb-Salze. Diese Salze werden bei allen Verfahren vor der hydrierung entfernt, insbesondere die Bleisalze, da Blei als starkes Katalysatorgift gilt (vgl. M. Freifelder, Practical Catalytic Hydrogenation, S. 24, 25, Wiley, New York (1971) und dort zitierte Lit.).

So ist in der US-Patentschrift 2 775 621 eine Bleientfernung über Ionenaustauscher vorgesehen, in den US-Patentschriften 2 276 192 und 2 271 083 werden die Katalysatorsalze vor der Hydrierung gefällt (z. B. als Sulfate). In der deutschen Patentschrift 830 951 (Seite 1, Zeile 7-23) werden die störenden Calcium- und Bleiionen als Carbonate gefällt, da sie bei der Hydrierung zu unerwünschten Nebenreaktionen führen und den Katalysator (Raney-Nickel) vergiften sollen. Man erreicht in diesem Fall zwar keine vollständige Entsalzung, es kann aber davon ausgegangen werden, daß das Blei relativ vollständig entfernt wird, wie ein Vergleich der Löslichkeitsprodukte zeigt :

$$CaCO_3 : 0{,}87 \cdot 10^{-8}\ (25\ °C)\quad PbCO_3 : 3{,}3 \cdot 10^{-14}\ (18\ °C)$$

In dem zitierten Patent wird die Restsalzlast (auf die Zusammensetzung wird nicht eingegangen) nach der Hydrierung durch Ionenaustauscher entfernt.

Die Reduktion von Formose an Raney-Nickel-Katalysatoren bei vorheriger Entfernung der Katalysatoren ist ferner aus folgenden Schriften bekannt : US 2 272 378 (Fällung von Pb mit $H_2SO_4$ oder $H_2S$) und DE-PS 888 096 (Fällung von Pb mit $H_2SO_4$).

Weitere Verfahren zur Reduktion von Formose unter Verwendung von Raney-Katalysatoren mit ein- oder zweistufiger Reaktionsführung sind in EP-A 0 002 473, EP-A 0 007 100 und 0 007 102 beschrieben, in

denen in den Beschreibungseinleitungen und in den Ausführungsbeispielen auf Formosen hingewiesen wird, denen die Katalysatorionen entzogen wurden. In den Ausführungsbeispielen wird jeweils eine Formose eingesetzt, die nach Beispiel 1 der DE-OS 27 21 186 gewonnen wurde. In diesem Beispiel 1 werden der Rohformose Metallionen und Säureanionen durch saure und basische Kationenaustauscher vollständig entzogen. Diese Entfernung der Metallionen ist insbesondere nötig für das Katalysatorgift Blei. Erst nach der Entsalzung findet eine für die katalytische Hydrierung erforderliche Einstellung des pH-Wertes statt, die u. a. mit $Ca(OH)_2$ vorgenommen werden kann. Ein eventuell vorhandener geringer Gehalt an Ca-Metall in den Raney-Skelettkatalysatoren ist fest in das Kristallgitter dieser Katalysatoren eingebaut und tritt als Ion in der zu hydrierenden Reaktionsmischung nicht auf.

In EP-A 0 002 473 wird weiterhin beschrieben, daß es notwendig ist, zur Hydrierung einen Gehalt an Carbonylgruppen von höchstens 2 Gew.-% des gesamten Reaktions-(Hydrier-) gemisches einzustellen. Um dies zu erreichen, wird zu einem vorgelegten Gemisch von Katalysator, komprimiertem Wasserstoff und Wasser oder bereits hydrierter Formose eine solche Menge noch nicht hydrierter Formose zugegeben, daß der Carbonylgehalt höchstens 2 Gew.-% dieses Gemisches beträgt ; sodann wird hydriert und ein solcher Anteil des hydrierten Gemisches abgezogen, daß ein weiterer Anteil noch nicht hydrierter Formose unter Einhaltung der genannten Bedingung wieder zugepumpt werden kann. Bei diesem chargenweisen Abziehen von hydrierter Formose, um noch nicht hydrierter Formose unter Einhaltung des Verdünnungseffektes Platz zu machen, verbleibt der Katalysator im Reaktor. Das genannte Verfahren ist sehr schwerfällig und langsam.

Es wurde nun ein Verfahren zur Herstellung eines Gemisches niedermolekularer Polyhydroxylverbindungen aus einem Gemisch von niedermolekularen Hydroxyaldehyden, Hydroxyketonen und Polyhydroxylverbindungen (Formose) durch katalytische Hydrierung bei 100 bis 200 bar Wasserstoffdruck an Nickel- oder Kobalt-haltigen Katalysatoren in einer Menge von 4 bis 240 Gew.-%, berechnet als Menge der aktiven Metalle und bezogen auf die im Hydrierreaktor vorliegende 100 %ige Formose, einem pH-Wert von 7,5 bis 12,5 und einer Temperatur von 80 bis 200 °C sowie gegebenenfalls in Gegenwart zugesetzter Aldehyde und/oder Ketone und/oder Alkohole und/oder Zucker, die nicht aus der Formose-Herstellung stammen, in einer Menge von bis zu 80 Gew.-%, bezogen auf die Gesamtmenge der zu reduzierenden Produkte, bei gleichzeitiger Entfernung des Gehaltes an Blei und gleichzeitiger Reduzierung des ursprünglichen Gehaltes an Calcium, wobei der Katalysator vorgelegt und gegebenenfalls vorhydriert wird, gefunden, das dadurch gekennzeichnet ist, daß man eine Rohformoselösung mit bis zu 70 Gew.-% Formose, die einen Gehalt an Bleiionen von 20 bis 16 000 ppm und/oder an Calciumionen von 9 770 bis 50 000 ppm, alles bezogen auf das Gesamtgewicht der Formoselösung, hat und gegebenenfalls noch andere Ionen von Metallen der I. und/oder II. Hauptgruppe des Periodensystems enthält, kontinuierlich dem vorgelegten Katalysator und dem ebenfalls vorgelegten komprimierten Wasserstoff zupumpt.

Gemische von niedermolekularen Hydroxyaldehyden, Hydroxyketonen und Polyhydroxylverbindungen (Formose) für das erfingungsgemäße Verfahren können beispielsweise nach einem der in der Beschreibungseinleitung angegebenen Verfahren hergestellt werden. Als Lösungsmittel für die Formosen sei beispielsweise Wasser oder ein Mono- oder Polyalkohol genannt, wie Methanol, Ethanol, Propanol, Butanol, Isopropanol, Isobutanol, Cyclopentanol, Cyclohexanol, 2-Ethoxyethanol, 2-Propoxyethanol, 2-Isopropoxyethanol, 2-Butoxyethanol, 2-(2-Methoxyethoxy)-ethanol, 2-(2-Ethoxyethoxy)-ethanol, 1,2-Bis-(2-hydroxyethoxy)-ethan, Ethylenglykol, Diethylenglykol, Triethylenglykol, Tetraethylenglykol, 1,2-Propandiol, Isopropylenglykol, 1,3-Propandiol, 1,2-Butandiol, 1,3-Butandiol, 1,4-Butandiol, 2-Methoxy-1-butanol, 2,3-Butandiol, 1,5-Pentandiol, 2,2-Dimethyl-1,3-propandiol, 1,6-Hexandiol, 2,5-Hexandiol, 2-Methyl-2,4-pentandiol, 3-Methyl-1,5-pentandiol, 3-Methyl-2,4-pentandiol, 2,3-Dimethyl-2,3-butandiol, 2-Methyl-2-propyl-1,3-propandiol, 2,2-Diethyl-1,3-propandiol, 2-Ethyl-1,3-hexandiol, 2,5-Dimethyl-2,5-hexandiol, 2,2,4-Trimethyl-1,3-pentandiol, 1,3-Diethoxy-2-propanol, 2-Hydroxymethyl-2-methyl-1,3-propandiol, 1,2,6-Hexantriol, 2-Ethyl-2-hydroxymethyl-1,3-propandiol, 2,2-Bis-hydroxymethyl-1,3-propandiol, Erythrit, Chinit, Mannit, Sorbit und Methylglykosit, sowie Ethoxylierungs- und Propoxylierungsprodukte dieser Alkohole mit einem Molekulargewicht bis etwa 400 und selbstverständlich auch Gemische dieser Alkohole. Bevorzugte Lösungsmittel sind Wasser, sowie Ethylenglykol, Glycerin und 1,4-Butandiol. Besonders bevorzugt als Lösungsmittel ist das Wasser.

Die Konzentration der Formose in einem der genannten Lösungsmittel kann bis zu 70 Gew.-%, bezogen auf die Gesamtlösung, betragen, beispielsweise 4 bis 70 Gew.-%, bevorzugt 20-70 Gew.-%, besonders bevorzugt 35 bis 70 Gew.-%.

Die Formoselösung kann weiterhin Aldehyde und/oder Ketone und/oder Alkohole und/oder Zucker, die nicht aus der Formoseherstellung stammen, in einer Menge bis zu 80 Gew.-%, bezogen auf die Gesamtmenge der zu reduzierenden Produkte, bevorzugt bis zu 30 Gew.-%, enthalten.

Als Aldehyde, die im erfindungsgemäßen Verfahren gegebenenfalls mitverwendet werden können, seien Beispielsweise Acetaldehyd, Propionaldehyd, Butyraldehyd, Isobutyraldehyd, sowie deren Methylolderivate, genannt.

Als Ketone, die im erfindungsgemäßen Verfahren gegebenenfalls mitverwendet werden können, seien beispielsweise Aceton, Methylethylketon, Diethylketon, Cyclopentanon, Cyclohexanon, Mesityloxid, Isophoron, Acetophenon, Benzophenon, sowie deren Methylolderivate, genannt.

Als Alkohole, die im erfindungsgemäßen Verfahren gegebenenfalls mitverwendet werden können,

seien außer den bereits als Lösungsmittel genannten Alkoholen noch Polyhydroxylverbindungen mit einem Molekulargewicht von bis 10 000 genannt, welche bei Raumtemperatur flüssig bzw. in der Formoselösung löslich sind, wie Polyester, Polyether, Polythioether, Polyacetale, Polycarbonate und Polyesteramide, die aus der Herstellung von Polyurethankunststoffen an sich bekannt sind und die mindestens 2, in der Regel 2 bis 8, vorzugsweise aber 2 bis 4, Hydroxylgruppen aufweisen.

Als Zucker, die im erfindungsgemäßen Verfahren gegebenenfalls mitverwendet werden können, seien beispielsweise künstliche oder auch natürliche Zucker genannt. Beispiele hierfür sind : Glucose, Maltose, Fructose, Saccharose, Lactose, künstliche Invertzucker, wie Hydrolysate von Rohrzucker, Mischungen von Rohrzucker und Invertzucker, Hydrolysate von Trehalose, Maltose oder Isomaltose, Hydrolysate von Mais- und Kartoffelstärke und Pektinstoffen, wie Amylose und Amylopektine, Cellobiose und Lactose, Hydrolysate von Galactose, Glucosemischungen, Raffinose-Hydrolysate, Cellulose-Hydrolysate, Hydrolysate von Dextrinen, gegebenenfalls in Mischung mit nicht hydrolysierten Dextrinen, Hydrolysate der Schardinger-Dextrine (cyclische Dextrine), Hydrolysate des Glycogens, Hydrolysate der Glucose-6-phosphorsäure, Hydrolysate von Glucose-1-phosphat (Cori-Ester), Fructose-6-phosphat, abgebaute Pektinstoffe (Polygalakturonsäuren), abgebaute Glucosamine, Hydrolysate von Melasserückständen.

Die genannten Aldehyde, Ketone, Alkohole und Zucker können einzeln oder in beliebigen Mischungen voneinander eingesetzt werden.

Erfindungsgemäß wird im beanspruchten Verfahren eine Formoselösung eingesetzt, die einen Gehalt an Bleiionen von bis 1,6 Gew.-% und/oder an Calciumionen von bis zu 5 Gew.-%, bezogen auf das Gesamtgewicht der Lösung, hat. Beispielsweise sei ein Gehalt an Bleiionen von 20 bis 16 000 ppm, bevorzugt 20 bis 2 000 ppm, besonders bevorzugt 100 bis 1 000 ppm, genannt. Weiterhin sei beispielsweise ein Gehalt an Calciumionen von 9 770 bis 50 000 ppm, bevorzugt 9 770 bis 40 000 ppm, besonders bevorzugt 9 770 bis 30 000 ppm, genannt. Formosen mit einem Gehalt an Blei- und/oder Calciumionen entstehen beispielsweise bei durch Blei- und/oder Calciumionen katalysierter Formaldehyd-Kondensation. Im erfindungsgemäßen Verfahren können demnach Formosen eingesetzt werden, die einen Gehalt an Bleiionen innerhalb des obengenannten Bereiches aufweisen. Ebenso können im erfindungsgemäßen Verfahren auch Formosen eingesetzt werden, die einen Gehalt an Calciumionen innerhalb des beschriebenen Bereiches aufweisen. Weiterhin können Formosen im erfindungsgemäßen Verfahren eingesetzt werden, die durch gemischte Katalyse mit Blei und Calcium-Ionen einen Gehalt an beiden Metallen innerhalb der genannten Bereiche aufweisen, wobei die Menge der beiden Ionenarten innerhalb der genannten Bereiche voneinander unabhängig sind. In bevorzugter Weise werden Formosen im erfindungsgemäßen Verfahren eingesetzt, die sowohl Blei- als auch Calciumionen enthalten.

Ein gegebenenfalls vorhandener Gehalt an anderen Ionen der I. und II. Hauptgruppe des Periodensystems in der Formose beeinträchtigt die Wirkungsweise des erfindungsgemäßen Verfahrens nicht. Solche Ionen sind beispielsweise die der Elemente Natrium, Kalium, Lithium, Magnesium, Strontium oder Barium.

Das erfindungsgemäße Verfahren wird in Gegenwart von Nickel- oder Kobalt-haltigen Katalysatoren durchgeführt. Solche Katalysatoren können in Form der reinen Elemente vorliegen, aber auch in Form von Trägerkatalysatoren und in Form von Legierungen oder Gemengen, die Nickel und/oder Kobalt enthalten.

Als Katalysatoren in Form der Elemente seien beispielsweise durch Reduktion von Nickel- oder Kobaltsalzen hergestellte Metallkatalysatoren, wie Urushibara-Nickel, oder mit Metallalkylverbindungen, Alkalihydriden, Hydrazin, Boranaten oder Borwasserstoff reduzierte Nikkel- oder Kobaltsalze, durch Reduktion der Metalloxide oder Metalloxidgemische hergestellte Katalysatoren genannt.

Als Träger für Nickel- und/oder Kobalt-haltige Katalysatoren seien sowohl anorganische Materialien, wie Kieselgur, Kieselsäure, Aluminiumoxide, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumkarbid, Aluminiumphosphat, Borphosphat, Asbest oder Aktivkohle, als auch organische Materialien, beispielsweise natürlich vorkommende oder synthetische Verbindungen mit hohem Molekulargewicht, wie Seide, Polyamide, Polystyrole, Zellstoff oder Polyurethane, genannt. Das Trägermaterial kann beispielsweise in Form von Kugeln, Strängen, Fäden, Zylindern, Polygonen oder in Pulverform vorliegen.

Als erfindungsgemäße Nickel- und/oder Kobalt-haltige Katalysatoren, die als Legierung oder Gemenge vorliegen, seien beispielsweise Katalysatoren vom Raney-Typ-gennant, wie Raney-Nickel, W-1-, W-5-, W-6- und W-7-Raney-Nickel (J. Am. Chem. Soc. *69*, 3039 (1974)), Raney-Kobalt-Katalysatoren, Raney-Nickel-Eisen, Raney-Kobalt-Nickel und Raney-Kobalt-Eisen. Die genannten Katalysatoren können als Promotoren eines oder mehrere der folgenden Elemente in Mengen bis zu 10 Gew.-% des Katalysatorgewichts enthalten : Lithium, Natrium, Calcium, Barium, Kalium, Silber, Beryllium, Lanthan, Cer, Vanadin, Niob, Tantal, Molybdän, Wolfram. Die genannten Katalysatoren können weiterhin eines oder mehrere der folgenden Elemente in Mengen bis zu 1 Gew.-% enthalten : Ruthenium, Rhodium, Palladium, Gold, Iridium, Platin.

In bevorzugter Weise werden für das erfindungsgemäße Verfahren Nickel-haltige Katalysatoren eingesetzt, wie Raney-Nickel, Raney-Nickel-Eisen, die gegebenenfalls noch Calcium und/oder Natrium enthalten können.

Der Katalysator wird im erfindungsgemäßen Verfahren in einer Menge von 4 bis 240 Gew.-%,

berechnet als Menge der aktiven Metalle und bezogen auf die im Hydrierreaktor vorliegende 100 %ige Formose, eingesetzt. In bevorzugter Weise wird eine Katalysatormenge von 6 bis 100, besonders bevorzugt 10 bis 40 Gew.-%, bezogen auf 100 %ige Formose, eingesetzt.

Das erfindungsgemäße Verfahren wird bei einem pH-Wert von 7,5 bis 12,5, bevorzugt 8,0 bis 10,5, durchgeführt. Der pH-Wert der zu hydrierenden Lösung kann sowohl mit anorganischen als auch mit organischen Basen eingestellt werden. Beispielsweise seien Natriumhydroxid, Kaliumhydroxid, Calciumhydroxid, Magnesiumhydroxid, Bariumhydroxid, Aluminiumoxidhydrat, Triethylamin, N-Methylmorpholin und N-Methylpiperidin genannt. Bevorzugt ist die Verwendung der bei der Formosesynthese bereits verwendeten Base. Für den Fall, daß bei der Formosesynthese Calciumhydroxid gleichzeitig als Katalysator und pH-Regler eingesetzt wurde, ist die Einstellung des pH-Wertes im erfindungsgemäßen Verfahren durch Calciumhydroxid besonders bevorzugt.

Das erfindungsgemäße Verfahren wird bei einem Wasserstoffdruck von 100 bis 200 bar und einer Temperatur im Bereich von 80 bis 200 °C, bevorzugt 100 bis 180 °C, besonders bevorzugt von 130 bis 180 °C, durchgeführt.

Das erfindungsgemäße Verfahren kann diskontinuierlich oder kontinuierlich durchgeführt werden. Hierbei können an sich bekannte Druckreaktoren, wie sie sonst für Hydrierungen üblich sind, gegebenenfalls mit einer geeigneten Rühr- oder Mischeinrichtung, verwendet werden. Der Katalysator kann hierbei fest angeordnet vorliegen oder als Suspension in der zu hydrierenden Formose gehalten werden. Diese letztere Variante ist vornehmlich bei der diskontinuierlichen Durchführung in einem Rühr- oder Schüttel-Reaktor oder bei der kontinuierlichen Durchführung in einem Rohrreaktor von Vorteil.

Bei der Durchführung des erfindungsgemäßen Verfahrens wird der Katalysator als Suspension in Wasser oder einem der weiter oben als Lösungsmittel genannten Alkohole vorgelegt. Sodann werden die gewünschte hydriertemperatur und der gewünschte Wasserstoffdruck eingestellt und der Katalysator durch eine Vorhydrierung aktiviert. Danach wird die zu hydrierende Formose in Form der weiter oben beschriebenen Lösung kontinuierlich bis zum zulässigen Füllvolumen in den Reaktor gepumpt, wobei die im Hydrierreaktor vorgelegte Katalysatormenge 4 bis 240 Gew.-%, bevorzugt 6 bis 100 Gew.-%, besonders bevorzugt 8 bis 35 Gew.-%, bezogen auf 100 %ige Formose, beträgt. Im Anschluß an die Zupumpzeit, läßt man noch während einer Zeit, die 3 bis 100 % der Zupumpzeit beträgt, nachhydrieren und drückt sodann den Gesamtinhalt aus dem Hydrierreaktor. Das Hydriergut wird sodann vom Katalysator abgetrennt, der zu wiederholten Malen für den gleichen Vorgang eingesetzt werden kann, wobei sich wiederum die Gesamtmenge Katalysator, bezogen auf die Gesamtmenge der in allen Hydrieransätzen reduzierten Formose, beträchtlich vermindert.

Es ist überraschend, daß im erfindungsgemäßen Verfahren « Roh-Formosen » eingesetzt werden können, die die gesamte Katalysatorsalzfracht an Bleiionen und/oder Calciumionen enthalten, während nach dem Stand der Technik solche Salzfrachten den Hydrierkatalysator innerhalb sehr kurzer Zeit desaktivieren. Dies gilt insbesondere für die Bleiionen, die als Katalysatorgift besonders wirksam sind. Es ist weiterhin überraschend, daß aus solchen Formosen ohne weitere Reinigung im erfindungsgemäßen Verfahren farblose Polyolgemische erhalten werden können. Weiterhin ist es überraschend, daß die zur Durchführung des erfindungsgemäßen Verfahrens eingesetzten Katalysatoren nicht nur bei ihrer erstmaligen Verwendung voll aktiv bleiben, sondern daß sie zu wiederholten Malen für das erfindungsgemäße Verfahren verwendet werden können. Diese wiederholte Einsetzbarkeit der Katalysatoren konnte nicht nur bei der Hydrierung von Formosen mit einer aus der Formose-Herstellung herrührenden üblichen Katalysatorsalzfracht beobachtet werden, sondern auch bei Formosen die zusätzlich zu der üblichen Katalysatorsalzfracht für Testversuche mit weiteren Mengen an Bleiionen oder an Calciumionen versetzt worden waren.

Ein ganz besonders überraschendes Merkmal ist jedoch die Tatsache, daß nicht nur der Katalysator in Gegenwart der beschriebenen Salzfracht seine Hydrieraktivität beibehält, sondern daß er zusätzlich die beschriebene Salzfracht in der zu hydrierenden Lösung reduziert. Es ist demnach ein weiteres Merkmal des erfinderischen Verfahrens, daß während der Hydrierung die Salzfracht der zu hydrierenden Lösung reduziert wird. Als Reduktion der Salzfracht in der zu hydrierenden Lösung sei beispielsweise eine Reduktion der Bleiionen auf einen Gehalt von weniger als 1 ppm genannt, so daß aus einer bleihaltigen Formose ein praktisch bleifreier formit wird. Als Reduktion der Salzfracht sei weiterhin beispielsweise eine Reduktion der Calciumionen auf weniger als 50 %, bevorzugt weniger als 30 % des ursprünglichen Wertes an Calciumionen genannt.

Obwohl der Vorgang der Reduktion der Bleiionen in der zu hydrierenden Formoselösung nicht völlig aufgeklärt ist, kann er offensichtlich als Einlegieren von nullwertigem Blei in das Katalysatormetall angesehen werden. Dieses Einlegieren des sonst als Katalysatorgift bekannten Bleis behindert den Einsatz des erfindungsgemäßen Katalysators zu wiederholten Malen nicht, so lange der Bleigehalt im Katalysator nicht den Wert von 30 Gew.-%, bevorzugt 20 Gew.-%, bezogen auf das ursprüngliche Gewicht des Katalysators, überschritten hat.

Die Reduktion des Gehaltes an Calciumionen in der zu hydrierenden Formose während des Hydriervorganges findet durch Ausfällung als Calciumcarbonat statt. Obwohl auch dieser Vorgang nicht völlig aufgeklärt ist, kann er als eine Zersetzung von vorhandenen Formiationen zu Kohlendioxid angesehen werden, das anschließend die Calciumionen in schwerlösliches Calciumcarbonat umwandelt und in dieser Form ausfällt. Auch von diesem Vorgang wird überraschenderweise die Aktivität des

Hydrierkatalysators nicht beeinträchtigt, so daß er zu wiederholten Malen eingesetzt werden kann. Es wird beobachtet, daß sich das ausgefällte Calciumcarbonat wenigstens teilweise auf dem Katalysator in Form von lose anhängenden Agglomeraten niederschlägt. Während, wie beschrieben, die katalytische Aktivität des Hydrierkatalysators, bezogen auf das Gewicht der aktiven Metalle, nicht absinkt, sinkt die spezifische Aktivität des infolge der Calciumcarbonat-Anlagerung im Gewicht zunehmenden Katalysator/Calciumcarbonat-Feststoffs, bezogen auf die Gesamtmasse an Feststoff. Es wird also bei wiederholtem Einsatz dieses mit dem Calciumcarbonat-Niederschlag versetzten Katalysators bei unverändertem Metallgehalt eine immer größere Feststoffmasse als Suspension im Hydriergemisch umgewälzt. Aus verfahrenstechnischen und damit wirtschaftlichen Gründen ist es daher sinnvoll, einen solchen Katalysator nur so lange wiederholt einzusetzen, wie sein Gehalt an ausgefälltem Calciumcarbonat den Wert von 100 Gew.-%, bevorzugt 70 Gew.-%, besonders bevorzugt 40 Gew.-%, an Calciumcarbonat, bezogen auf das ursprüngliche Gewicht des Katalysators, nicht überschreitet.

Überraschenderweise wurde weiterhin gefunden, daß der Calciumcarbonat-Niederschlag infolge seiner nur geringen mechanischen Bindung an den Hydrierkatalysator dadurch entfernt werden kann, daß man den mit Calciumcarbonat belegten Katalysator bei einem beliebigen Belegungsgrad mit Calciumcarbonat in Wasser oder einem anderen das Calciumcarbonat nicht lösenden flüssigen Medium, bevorzugt aber in Wasser, als Suspension so kräftig aufrührt, daß die lose anhaftenden Calciumcarbonatteilchen von den Katalysatorteilchen getrennt werden. In einer nachfolgenden Beruhigungsphase, beispielsweise durch Abschalten des Rührers, setzen sich sodann die Katalysatorteilchen infolge ihrer größeren Dichte rasch am Boden des Gefäßes ab, so daß das noch in Suspension befindliche Calciumcarbonat abdekantiert werden kann. Der so vom Calciumcarbonat-Niederschlag befreite Katalysator zeigt keine Einbuße seiner katalytischen Aktivität und kann, wie weiter oben beschrieben, zu wiederholten Malen in dem erfindungsgemäßen Verfahren eingesetzt werden.

Das erfindungsgemäße Verfahren zeichnet sich durch folgende technisch fortschrittlichen Merkmale aus :

1. Der Einsatz von « Roh-Formose », die die gesamte Katalysatorsalzfracht an Blei- und/oder Calciumionen enthält, ist möglich. Eine nach dem Stande der Technik für erforderlich gehaltene Reduzierung der genannten Ionen vor der Hydrierstufe ist demnach nicht erforderlich.

2. Der Katalysatoraufwand ist infolge der Aufrechterhaltung seiner Aktivität und seines wiederholten Einsatzes trotz der Beladung mit den Umwandlungsprodukten der Blei- und/oder Calciumionen sehr gering.

3. Im erfindungsgemäßen Verfahren können Formite erhalten werden, die bleifrei und arm an Calciumionen sind. Diese Formite können für viele Verwendungszwecke ohne weitere Reinigung eingesetzt werden.

4. Die Hydrierung im erfindungsgemäßen Verfahren läuft schneller ab als die Hydrierung nach Verfahren des Standes der Technik. Insbesondere die Verfahrensvariante, bei der zu einem vorgelegten und aktivierten Katalysator und ebenfalls vorgelegtem komprimierten Wasserstoff die Formose kontinuierlich bis zum zulässigen Kesselinhalt zugepumpt werden kann, erlaubt sehr kurze Hydrierzeiten.

5. Durch die bekannte Spaltung langkettiger Formoseanteile (DE-OS 27 56 274), die durch die vorhandene Salzlast noch verstärkt wird, werden niedermolekulare Polyalkohole erhalten. Ein Maß hierfür ist die durchschnittliche Hydroxyl-Funktionalität des Hydriergutes, wobei im allgemeinen jedes C-Atom der erhaltenen Polyalkohole eine OH-Gruppe trägt.

6. Das erhaltene Polyolgemisch ist farblos. Solche Polyolgemische, die im erfindungsgemäßen Verfahren durch Mitverwendung von Aldehyden, Ketonen oder Polyolen, besonders höhermolekularer Polyole, erhältlich sind, zeigen eine verbesserte Verträglichkeit dieser Gemische gegenüber den im Polyisocyanat-Poly-additions-Verfahren verwendeten Treibmitteln.

Die erfindungsgemäß herstellbaren Polyolgemische (Formite) sind vielfältig verwendbar. So können sie unmittelbar als Polyol-Kettenverlängerer und/oder -Vernetzer bei der Herstellung von Polyurethan-Kunststoffen eingesetzt werden. Sie können aber auch als Ausgangsstoffe zur Herstellung von Polyethern oder Polyestern dienen, die ihrerseits für die Herstellung von Polyurethankunststoffen, aber auch anderen Kunststoffen, beispielsweise Alkydharzen, verwendet werden können. Weiterhin können die erfindungsgemäß hergestellten Formite als Gefrierschutzmittel oder als Formulierhilfsmittel auf dem Pflanzenschutzsektor dienen.

## Beispiel 1

(Vergleichsbeispiel « Batch-Hydrierung »)

In einem 3 l Autoklav werden 44 g Raney-Nickel in 400 ml Wasser vorgelegt und 45 Minuten bei 140 °C und 150 bar $H_2$-Druck vorhydriert. Man läßt abkühlen und gibt, ohne zu entspannen, 1 845 g (= 1,5 l) Formose-Lösung gemäß Beispiel 2 II aus DE-OS 2 721 186 (47,8 Gew.-%ig, bleifrei ; mit $Ca(OH)_2$ auf pH = 9,0 eingestellt) hinzu. Die Temperatur wird auf 80 °C erhöht und bei 150 bar Wasserstoffdruck wird 5 Stunden hydriert, wobei die Temperatur jede Stunde um 20 °C gesteigert wird, bis 140 °C erreicht

sind. Bei dieser Temperatur läßt man die restliche Zeit nachhydrieren. Danach wird die Lösung abgedrückt, filtriert und analysiert. Das schwarz-braune Filtrat hat einen pH-Wert von 6,2 und färbt die Fehlingsche Lösung rot, besitzt also noch reduzierende Anteile.

Beispiel 1 zeigt, daß eine Batch-Hydrierung, wie sie z. B. bei der Reduktion von D-Glucose zu Sorbit angewendet wird, auf Formose nicht ohne weiteres übertragen werden kann.

Beispiele 2 bis 9

(« Kontinuierliche Pumphydrierung »)

Allgemeine Versuchsbeschreibung

Der Katalysator Raney-Nickel/Eisen (85/15) wird in wenig Wasser im Autoklaven vorgelegt und bei 140 °C und 150 bar $H_2$-Druck 45 Minuten aktiviert.

Die Formoselösungen (Rohprodukte mit Salzlast) gemäß Beispiel 1 aus DE-OS 2 738 512 (für Ausführungsbeispiel 2), gemäß Beispiel 1 aus DE-OS 2 721 186 (für Ausführungsbeispiel 7) und gemäß 2 II aus DE-OS 2 721 186 (für Ausführungsbeispiele 3-6, 8 und 9) werden mit $Ca(OH)_2$ auf die gewünschte Alkalität eingestellt und anschließend die für den jeweiligen Autoklaventyp vorgegebene Betriebsmenge kontinuierlich so in den Reaktor gepumpt, daß der Druck nicht unter 150 bar und die Temperatur nicht unter 140 °C abfallen. Man hydriert etwa 1/10 der Pumpzeit nach und drückt dann ab. Das nach dem Abtrennen des Katalysators farblose Polyolgemisch (Formit) wird analysiert und kann gegebenenfalls im Vakuum vom Wasser bzw. durch schwefelsaure Fällung von noch vorhandenem Calcium befreit werden. Die angegebene mittlere OH-Funktionalität (Komponentenverteilung) wurde aus der gaschromatographischen Analyse berechnet.

(Siehe Tabelle, Seite 8 f.)

| Beispiel Nr. | Autoklav (l) | Formose (kg) | Konz. (%) | $Ca^{2+}$ [ppm] | $Pb^{2+}$ [ppm] | Katalysator * (g) | pH nach Einstellung mit $Ca(OH)_2$ | Gesamthydrierzeit [h] | Restgehalt Carbonyl (%) | Restgehalt Ca 2+ [ppm] | Restgehalt Pb 2+ [ppm] | mittlere Funktionalität |
|---|---|---|---|---|---|---|---|---|---|---|---|---|
| 2 | 3 | 1,735 | 48 | 26210 | - | 133 | 9,0 | 7 | 0,008 | 4600 | - | 4,9 |
| 3 | 3 | 1,735 | 48 | 10450 | 431 | 133 | 9,0 | 7 | 0,008 | 3600 | < 1 | 3,9 |
| 4 | 3 | 1,735 | 68,3 | 9940 | 620 | 133 | 9,1 | 7 | 0,006 | 3300 | < 1 | 3,9 |
| 5 | 3 | 1,735 | 36,6 | 12100 | 260 | 133 | 9,1 | 1,75 | 0,03 | 1800 | < 1 | 4,2 |
| 6 | 3 | 1,735 | 47,8 | 10000 | 15330*** | 133 | 8,6 | 7 | 0,03 | 4200 | < 1 | 4,0 |
| 7 | 3 | 1,735 | 45,5 | -** | 923 | 133 | 9,1** | 7 | 0,004 | - | < 1 | 4,1 |
| 8 | 1800 | 1042 | 47,8 | 9770 | - | 80000 | 9,0 | 7 | 0,04 | 2250 | - | 4,0 |
| 9 | 1800 | 966 | 65 | 9990 | 620 | 80000 | 9,0 | 7 | 0,006 | 2000 | < 1 | 4,0 |

* Für die Beispiele 2 bis 7 und 8 und 9 wurde der Katalysator stets wiederverwendet.
** Wurde mit NaOH alkalisch gestellt : Na-Gesamtgehalt : 5 600 ppm.
*** Enthält nachträglich zugesetzte Pb-Ionen.

## Ansprüche

1. Verfahren zur Herstellung eines Gemisches niedermolekularer Polyhydroxylverbindungen aus einem Gemisch von niedermolekularen Hydroxyaldehyden, Hydroxyketonen und Polyhydroxylverbindungen (Formose) durch katalytische Hydrierung bei 100-200 bar Wasserstoffdruck an Nickel- oder Kobalt-haltigen Katalysatoren in einer Menge von 4 bis 240 Gew.-%, berechnet als Menge der aktiven Metalle und bezogen auf die im hydrierreaktor vorliegende 100 %ige Formose, einem pH-Wert von 7,5 bis 12,5 und einer Temperatur von 80-200 °C sowie gegebenenfalls in Gegenwart zugesetzter Aldehyde und/oder Ketone und/oder Alkohole und/oder Zucker, die nicht aus der Formose-herstellung stammen, in einer Menge von bis zu 80 Gew.-%, bezogen auf die Gesamtmenge der zu reduzierenden Produkte, bei gleichzeitiger Entfernung des Gehaltes an Blei und gleichzeitiger Reduzierung des ursprünglichen Gehaltes an Calcium, wobei der Katalysator vorgelegt und gegebenenfalls vorhydriert wird, dadurch gekennzeichnet, daß man eine Rohformoselösung mit bis zu 70 Gew.-% Formose, die einen Gehalt an Bleionen von 20 bis 16 000 ppm und/oder an Calciumionen von 9 770 bis 50 000 ppm, alles bezogen auf das Gesamtgewicht der Lösung, hat, und gegebenenfalls andere Ionen von Metallen der I. und II. Hauptgruppe des Periodensystems enthält, kontinuierlich dem vorgelegten Katalysator und dem ebenfalls vorgelegten komprimierten Wasserstoff zupumpt.

2. Verfahren nach Anspruch 1, dadurch gekennzeichnet, daß man den Katalysator so lange wiederverwendet, wie sein Gehalt an Blei den Wert von 30 Gew.-%, bezogen auf das ursprüngliche Gewicht des Katalysators, nicht überschreitet.

3. Verfahren nach Anspruch 1, dadurch gekennzeichnet, daß man den Katalysator so lange wiederverwendet, wie sein Gehalt an niedergeschlagenem Calciumcarbonat den Wert von 100 Gew.-%, bezogen auf das ursprüngliche Gewicht des Katalysators, nicht überschreitet.

4. Verfahren nach Anspruch 3, dadurch gekennzeichnet, man den Katalysator vom niedergeschlagenen Calciumcarbonat durch kräftiges Aufrühren des mit Calciumcarbonat belegten Katalysators, Absitzenlassen der schweren Katalysatorteilchen und Abdenkantieren der schwebenden Calciumcarbonat-Teilchen befreit und danach wiederverwendet.

## Claims

1. Process for the preparation of a mixture of low-molecular polyhydroxy compounds from a mixture of low-molecular hydroxyaldehydes, hydroxyketones and polyhydroxy compounds (formose) by catalytic hydrogenation at a hydrogen pressure of 100-200 bars on nickel-containing or cobalt-containing catalysts in a quantity of 4 to 240 % by weight, calculated as the amount of the active metals and based on the 100 % formose present in the hydrogenation reactor, a pH value of 7.5 to 12.5 and a temperature of 80-200 °C and if appropriate in the presence of added aldehydes and/or ketones and/or alcohols and/or sugars which do not originate from the preparation of the formose, in a quantity of up to 80 % by weight, based on the total quantity of the products to be reduced, with simultaneous removal of the content of lead and simultaneous reduction of the original content of calcium, the catalyst being initially introduced and optionally pre-hydrogenated, characterised in that a crude formose solution which contains up to 70 % by weight of formose and has a content of lead ions of 20 to 16,000 ppm and/or of calcium ions of 9,770 to 50,000 ppm, in each case based on the total veight of the solution, and which may contain other ions of metals of main groups I and II of the periodic system, is continuously pumped into the initially introduced catalyst and the compressed hydrogen which has also been initially introduced.

2. Process according to Claim 1, characterised in that the catalyst is re-used for as long as its content of lead does not exceed the value of 30 % by weight, based on the original weight of the catalyst.

3. Process according to Claim 1, characterised in that the catalyst is re-used for as long as its content of precipitated calcium carbonate does not exceed the value of 100 % by weight, based on the original weight of the catalyst.

4. Process according to Claim 3, characterised in that the catalyst is freed from the precipitated calcium carbonate by vigorously stirring the catalyst coated with calcium carbonate, allowing the heavy catalyst particles to settle and decanting off the suspended calcium carbonate particles and the catalyst is then re-used.

## Revendications

1. Procédé pour la fabrication d'un mélange de composés polyhydroxylés de bas poids moléculaire à partir d'un mélange d'hydroxyaldéhydes, d'hydroxyacétones et de composés polyhydroxylés (formoses) de bas poids moléculaire par hydrogénation catalytique sous une pression d'hydrogène de 100 à 200 bars sur des catalyseurs au nickel ou au cobalt en quantité de 4 à 240 % en poids, calculée comme quantité des métaux actifs et rapportée au formose à 100 % présent dans le réacteur d'hydrogénation, à un pH de 7,5 à 12,5, et une température de 80 à 200 °C et éventuellement en présence d'aldéhydes et/ou de cétones et/ou d'alcools et/ou de sucres ajoutés qui ne proviennent pas de la fabrication du formose, en quantité allant

jusqu'à 80 % en poids, par rapport à la quantité totale des produits à réduire, avec élimination simultanée de la teneur en plomb et réduction simultanée de la teneur initiale en calcium, le catalyseur étant préalablement chargé et éventuellement préalablement hydrogéné, caractérisé en ce que l'on ajoute en continu par pompage au catalyseur préalablement chargé et à l'hydrogène comprimé préalablement chargé également une solution de formose brut contenant jusqu'à 70 % en poids de formose, qui contient 20 à 16 000 ppm d'ions plomb et/ou 9 770 à 50 000 ppm d'ions calcium, par rapport au poids total de la solution de formose, et qui contient éventuellement encore d'autres ions de métaux du groupe principal I et/ou du groupe principal II de la Classification Périodique.

2. Procédé selon la revendication 1, caractérisé en ce que l'on réutilise le catalyseur tant que sa teneur en plomb ne dépasse pas la valeur de 30 % en poids, par rapport au poids initial du catalyseur.

3. Procédé selon la revendication 1, caractérisé en ce que l'on utilise le catalyseur tant que sa teneur en carbonate de calcium précipité ne dépasse pas la valeur de 100 % en poids, par rapport au poids initial du catalyseur.

4. Procédé selon la revendication 3, caractérisé en ce que l'on débarrasse le catalyseur du carbonate de calcium précipité en agitant vigoureusement le catalyseur chargé de carbonate de calcium, en laissant déposer les particules lourdes de catalyseur et en séparant par décantation les particules flottantes de carbonate de calcium et on réutilise le catalyseur.